# EUROPEAN PATENT APPLICATION

(11) **EP 2 682 005 A1**
(43) Date of publication of application: **08.01.2014**
(21) Application number: 12175151.5
(22) Date of filing: 05.07.2012
(51) Int. Cl.: A23L 1/30, A61K 31/35, A61K 31/352, A61K 31/7048, A61K 36/73, A61K 36/87, A61K 36/45, A61P 39/06

(54) **A dietary supplement composition**

(71) Applicant: Symrise AG, 37603 Holzminden (DE)
(72) Inventor: Stürtz, Melanie, 34385 Bad Karlshafen (DE); Roloff, Michael, 34399 Oberweser (DE); Herbst, Hans-Thomas, 37696 Marienmünster (DE); Erfurt, Harry, 37170 Uslar (DE); Niemeyer, Hans-Jürgen, 37603 Holzminden (DE)
(74) Representative: Fabry, Bernd

(57) **Abstract**

Suggested is a dietary supplement composition comprising one or two anthocyanins, wherein the anthocyanin radical of said anthocyanins are essentially selected from the group consisting of
(a) Delphinidin and/or
(b) Cyanidin,

and the corresponding sugar radicals are essentially selected from the group consisting of glucose, galactose, xylose, arabinose, rutinose, sophorose, sambubiose, or their mixtures,
wherein
(i) the content of disaccharides is less than about 2 % b.w. and
(ii) optionally the content of potassium is about 10 to 5.000 ppm
- both calculated on the composition.

## Description

### Field of invention

The present invention belongs to the area of nutrition and refers to new dietary supplement compositions comprising selected mixtures of anthocyanins showing improved anti-oxidative and anti-inflammatory activities.

### State of the art

A dietary supplement, also known as food supplement or nutritional supplement, is a preparation intended to supplement the diet and provide nutrients, such as vitamins, minerals, fibres, fatty acids, or amino acids, that may be missing or may not be consumed in sufficient quantities in a person's diet. Some countries define dietary supplements as foods, while in others they are defined as drugs or natural health products.

Nowadays the term dietary supplement is also used for any composition for oral uptake that is intended to improve the over-all health status of a human. Probiotic micro-organisms, prebiotics and in particular various plant extracts are the most common actives one can find in the market. Especially flavonoids in general and anthocyans in particular have gained more and more importance due to their toxicological safety and ― inter alia ― high anti-oxidative power. Anthocyanidins and their glycosides, called anthocyanins, can be found in various red, blue and black fruits, as for example blackberries, red and black current, but also in blossoms, like for example hibiscus. Although hundreds of different anthocyanins are known, the "big five" ― delphinidin, cyanidin, petunidin, peonidin and malvidin ― are found as glycosides in most of its biological sources.

The usefulness of these products is very well known. For example, Kähkönen et al. have investigated the anti-oxidative activity of about 100 plant extracts [J. Agric. Food Chem., 47, p 3954-3962 (1999**)].** Zheng et al. report about the oxygen radical absorbing capacity of anthocyanins extracted from various berries [J. Agric. Food. Chem., 51, p 502-509 (2003**)].** Ogawa et al. have studied the anti-oxidative properties of crowberries and other natural sources and have identified their anthocyanin composition [J. Agric. Food. Chem., 56, p 4457-4462 (2008**)].** A similar study has been conducted by Riedl et al. [Ernährung/Nutrition, 36, p 464-469 (2011**)].**

All this research work has clearly proven the excellent properties of anthocyanins obtained from natural sources to scavenge radicals and to avoid cell damages caused by oxygen or UV radiation. However, the state of the art also shows that the performance of anthocyanin compositions differs seriously by nature of origin and ― consequently ― by composition of the main constituents.

Therefore, the object of the present invention has been to develop new advantageous anthocyanin compositions, so that the beneficial properties of the actives, namely their anti-oxidative power and anti-inflammatory activity are significantly increased.

### Description of the invention

Object of the present invention is a dietary supplement composition comprising one or two anthocyanins, wherein the anthocyanin radical of said anthocyanins are essentially selected from the group consisting of
(a) Delphinidin and/or
(b) Cyanidin,
   and the corresponding sugar radicals are essentially selected from the group consisting of glucose, galactose, xylose, arabinose, rutinose, sophorose, sambubiose, or their mixtures,
   wherein
   (i) the content of disaccharides is less than about 2 % b.w. and
   (ii) optionally the content of potassium is about 10 to 5.000 ppm
      - both calculated on the composition.

Surprisingly it has been observed that delphinidin and cyanidin, either taken alone or in a binary mixture, show superior anti-oxidative and anti-inflammatory performance when compared to standard preparations comprising also petunidin, peonidin and malvidin. The compositions according to the present invention also show low contents of sugar and are preferably rich in potassium, which is also of benefit for consumption and the overall health status of a human.

### Preparation and composition of the anthocyanins

### A. Anthocyanins

Anthocyanins represent water-soluble vacuole pigments that may appear red, purple, or blue according to the pH. They belong to the class of flavonoids synthesized via the phenylpropanoid pathway following general formula (I)

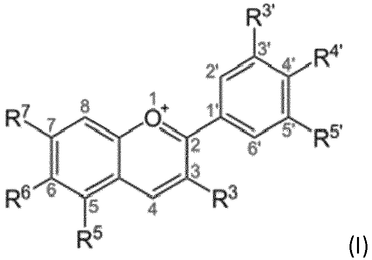

Anthocyanins are odourless and nearly flavourless, contributing to taste as a moderately astringent sensation. Anthocyanins occur in all tissues of higher plants, including leaves, stems, roots, flowers, and fruits. Anthoxanthins are their clear, white to yellow counterparts occurring in plants. Anthocyanins are derivatives of anthocyanidins, which include pendant sugars. Most frequent in nature are the glycosides of cyanidin, delphinidin, malvidin, pelargonidin, peonidin, and petunidin. Roughly 2% of all hydrocarbons fixated in photosynthesis are converted into flavonoids and their derivatives such as the anthocyanins. **Table A** provides an overview of the most common anthocyanin species.

**Table A**

| Anthocyanins | | | | | | | | |
|---|---|---|---|---|---|---|---|---|
| **Anthocyanin** | **Basic structure** | **R_{3'}** | **R_{4'}** | **R_{5'}** | **R₃** | **R₅** | **R₆** | **R₇** |
| Aurantinidin | 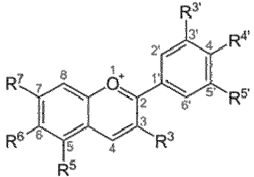 | -H | -OH | -H | -OH | -OH | -OH | -OH |
| Cyanidin | | -OH | -OH | -H | -OH | -OH | -H | -OH |
| Delphinidin | | -OH | -OH | -OH | -OH | -OH | -H | -OH |
| Europinidin | | -OCH₃ | -OH | -OH | -OH | -OCH₃ | -H | -OH |
| Luteolinidin | | -OH | -OH | -H | -H | -OH | -H | -OH |
| Pelargonidin | | -H | -OH | -H | -OH | -OH | -H | -OH |
| Malvidin | | -OCH₃ | -OH | -OCH₃ | -OH | -OH | -H | -OH |
| Peonidin | | -OCH₃ | -OH | -H | -OH | -OH | -H | -OH |
| Petunidin | | -OH | -OH | -OCH₃ | -OH | -OH | -H | -OH |
| Rosinidin | | -OCH₃ | -OH | -H | -OH | -OH | -H | -OCH₃ |

The anthocyanins mostly represent 3-O-glycosides. The most relevant aglycons are those formed from glucose, galactose and arabinose, however also sambubiosides, sophorosides and rutinosides occur.

As of 2003 more than 400 anthocyanins had been reported while more recent literature (early 2006), puts the number at more than 550 different anthocyanins. The difference in chemical structure that occurs in response to changes in pH is the reason why anthocyanins are often used as pH indicator.

Anthocyanins are thought to be subject to physiochemical degradation in vivo and in vitro. It is generally known that structure, pH, temperature, light, oxygen, metal ions, intramolecular association, and intermolecular association with other compounds (co-pigments, sugars, proteins, degradation products, etc.) are known to affect the colour and stability of anthocyanins. It has been demonstrated that B-ring hydroxylation status and pH mediate the degradation of anthocyanins to their phenolic acid and aldehyde constituents. Indeed, significant portions of ingested anthocyanins are likely to degrade to phenolic acids and aldehyde in vivo, following oral consumption. This characteristic confounds scientific isolation of specific anthocyanin mechanisms in vivo.

Cancer research on anthocyanins is the most advanced, where black raspberry (*Rubus occidentalis* L.) preparations were first used to inhibit chemically induced cancer of the rat esophagus by 30-60% and of the colon by up to 80%. Effective at both the initiation and promotion/progression stages of tumour development, black raspberries are a practical research tool and a promising therapeutic source, as they contain the richest contents of anthocyanins among native North American Rubus berries. Work on laboratory cancer models has shown that black raspberry anthocyanins inhibit promotion and progression of tumour cells by
- stalling growth of pre-malignant cells;
- accelerating the rate of cell turnover, called apoptosis, effectively making the cancer cells die faster;
- reducing inflammatory mediators that initiate tumour onset;
- inhibiting growth of new blood vessels that nourish tumours - a process called angiogenesis; and
- minimising cancer-induced DNA damage.

On a molecular level, berry anthocyanins were shown to turn off genes involved with tumour proliferation, inflammation and angiogenesis, while switching on apoptosis. In 2007, black raspberry studies entered the next pivotal level of research ― the human clinical trial ― for which several approved studies are underway to examine anti-cancer effects of black raspberries and cranberries on tumours in the esophagus, prostate and colon.

In a preferred embodiment of the present invention the anthocyanins are obtained from extracts and/or juices from red berries or red blossoms, selected from the group consisting of:
- **Açai fruits** (*Euterpe oleracea*);
- **Aronia** (e.g. *Aronia arbutifolia, Aronia melanocarpa; Aronia prunifolia*);
- **Black and Red currents** (e.g. *Ribes rubrum, Ribes spciatum, Ribes alpinum, Ribes schlechtendalii, Ribes multiflorum, Ribes petraeum, Ribes trite, Ribes nigrum);*
- **Blackberries** (*Rubus sp*.);
- **Black Carrots** (*Daucus carota*);
- **Black Tomatoes** (*Solanum lycopersicum*);
- **Blood oranges** (*Citrus sinensis*);
- **Blueberries** (*Vaccinium corymbosum, Vaccinium angustifolium*);
- **Blackthorn** (*Prunus spinosa*);
- **Bog Bilberries** (*Vaccinium ulginosum*);
- **Cloudberries** (*Rubus chamaemorus*);
- **Cranberries** (e.g. *Vaccinium oxycoccos, Vaccinium microcarpus, Vaccinium macrocarpus, Vaccinium erythrocarpus*);
- **Crowberries** (e.g. *Empetrum nigrum, Empetrum eamesii, Empetrum rumbrum, Empetrum hermaphroditum*);
- **Elderberries** (e.g. *Sambucus nigra, Sambucus racemosa*);
- **Hibiscus** (*Hibiscus sabdariffa, Roselle*);
- **Lingonberries** (e.g. *Vaccinium vitus idaea*);
- **Magellan Barberries** ("Calafate", *Berberis microphylla, Breberis buxifolia*);
- **Maqui berries** (e.g. *Aristotelia chilensis*);
- **Mountain Bilberries** (*Vaccinium membranaceum*);
- **Prunes and prune plums** (*Prunus domestica*);
- **Raspberry** (*Rubus idaeus, Rubus occidentalis);*
- **Red Gooseberries** (*Ribes uva-crispa, Ribes grossularia*);
- **Red grapes** (*Vitis vinifera, Vitis labrusca, Vitis riparia, Vitis rotundifolia*);
- **Rowanberries** (*Sorbus aucuparia*);
- **Sour cherries** (e.g. *Prunus avium, Prunus cerasus);*
- **Strawberries** (*Fragaria ananassa*);
- **Sweet Cherries** (*Prunus avium*);
- **Tayberries** (*Rubus X*).

It should be noted that for example elderberries, aronia and red current contain only anthocyanins based on cyanidin. In all other cases it is preferred, that the ratio by weight between delphinidin and cyanidin is in the range of about 1 : 3 to about 3 : 1, and preferably about 1 : 2 to 2 : 1.

The composition may also encompass least one carboxylic acid selected from the group consisting of phenolic acids, as for example gallic acid, sinapic acid, procatechuic acid, cholorogenic acid and/or their esters. Phenolic acids, and in particular hydroxycinnamic acids or hydroxybenzoeic acids show various advantageous activities as for example:
- decrease the uptake of glucose in the body and assist in regulating blood glucose level;
- impact in the reduction of lipid peroxidation resulting in significant heart health;
- antioxidant, free radical scavenging and metal chelating properties;
- potential health-promoting effects: anti-inflammatory, enzyme inhibition, antimicrobial, vascular and cytotoxic antitumor activity;
- protection against oxidative damage diseases (coronary heart disease, stroke, and cancer);
- support of weight loss;
- strong inhibition of tyrosine nitration by per nitrate;
- role in the enhancement of insulin action and glucose uptake in the bloodstream;
- helps to boost metabolism and increase the amount of weight loss (for overweight individuals);
- consumption of high doses increase plasma total homocysteine concentration in humans;

In particular, procatechuic acid is known for in vitro inhibitory activity on beta-glucosidase, anti-oxidative, anti-bacterial and anti-mutagenic activity. It is also discussed as a potential cancer chemo-preventive agent and may be used in medicine to prevent cardiovascular diseases and cancer. In fact, procatechuic acid is a major metabolite of cyanidin-glucosides.

It is also preferred that the content of disaccharides in compositions is less than about 2 % b.w., more preferably about 0.1 to about 1.5 % b.w. ― calculated on the mixture.

Typically, and in a preferred embodiment of the invention, the compositions are rich in potassium and contain the element in the form of salts in concentration of about 10 to about 5.000, preferably about 100 to about 2.500 and more preferably about 200 to about 1.000 ppm.

### B. Preparation

How to obtain a juice from a berry belongs to common art. The extracts according to the present invention may be prepared by methods known per se, i.e. for example by aqueous, alcoholic or aqueous/alcoholic extraction of the plants or parts thereof or shells or tresters of the plants. Suitable extraction processes are any conventional extraction processes, such as maceration, re-maceration, digestion, agitation maceration, vortex extraction, ultrasonic extraction, counter current extraction, percolation, re-percolation, evacolation (extraction under reduced pressure), diacolation and solid/liquid extraction under continuous reflux. Percolation is advantageous for industrial use. Leaves, fruits, blossoms or roots represent suitable starting materials and may be mechanically size-reduced before the extraction process. Any size reduction methods known to the expert, for example freeze grinding, may be used. Preferred solvents for the extraction process are organic solvents, water (preferably hot water with a temperature above 80 °C and more particularly above 95 °C or mixtures of organic solvents and water, more particularly low molecular weight alcohols, preferably ethanol, with more or less high water contents. Extraction with food grade ethanol and water-containing mixtures thereof is particularly preferred. In case temperature sensitive molecules are to be extracted, steam distillation is the preferred method (for example in case of hibiscus blossoms or the preparation of citrus oils).

The extraction process is generally carried out at about 20 to about 100 °C and preferably at about 50 to about 70 °C. In one preferred embodiment, the extraction process is carried out in an inert gas atmosphere to avoid oxidation of the ingredients of the extract. This is particularly important where extraction is carried out at temperatures above 40 °C. The extraction times are selected by the expert in dependence upon the starting material, the extraction process, the extraction temperature and the ratio of solvent to raw material, etc.

A preferred process for obtaining the extracts is a combination of liquid-liquid extraction and adsorption using a polymer ion exchanger resin as it is for example generically described in WO 2003 043646 A1 (Cognis).

After the extraction process, the crude extracts obtained may optionally be subjected to other typical steps, such as for example purification, concentration and/or decolouration. If desired, the extracts thus prepared may be subjected, for example, to the selective removal of individual unwanted ingredients. The extraction process may be carried out to any degree, but is usually continued to exhaustion. Typical yields (=extract dry matter, based on the quantity of raw material used) in the extraction of the starting materials are of the order of about 1 to about 20, %, preferably about 2 to about 15 and more preferably about 5 to about 10 % b.w. - calculated on the starting materials.

### Additives

The dietary supplement compositions according to the present invention may include the mixtures of anthocyanins along with at least one additive selected from the group consisting of (b1) probiotic micro-organisms, (b2) prebiotics, (b3) vitamins and (b4) minerals.

### A. Probiotic micro-organisms

Probiotic organisms, also called "probiotics" forming component (b1) represent live micro-organisms which are considered to be beneficial to the host organism. According to the currently adopted definition by FAO/WHO, probiotics are: "Live microorganisms which when administered in adequate amounts confer a health benefit on the host". Lactic acid bacteria (LAB) and bifidobacteria are the most common types of microbes used as probiotics; but certain yeasts and bacilli may also be used. Probiotics are commonly consumed as part of fermented foods with specially added active live cultures; such as in yogurt, soy yogurt, or as dietary supplements. Live probiotic cultures are available in fermented dairy products and probiotic fortified foods. However, tablets, capsules, powders and sachets containing the bacteria in freeze dried form are also available. Table B provides an overview of common probiotics and their respective health claims that can be used as component (b1) of the present invention:

**Table B**

| Probiotics | | | |
|---|---|---|---|
| **Strain** | **Brand name** | **Producer** | **Health claims** |
| *Bacillus coagulans* GBI-30, 6086 | GanedenBC | Ganeden Biotech | May improve abdominal pain and bloating in IBS patients. May increase immune response to a viral challenge. |
| *Bifidobacterium animalis* subsp. *lactis* BB-12 | Probio-Tec Bifidobacterium BB-12 | Chr. Hansen | Human studies have shown that BB-12 alone or in combination may have an effect on the gastrointestinal system. |
| *Bifidobacterium infantis* 35624 | Align | Procter & Gamble | In one preliminary study, showed possible improvement for abdominal pain/discomfort and bowel movement difficulty. |
| *Lactobacillus acidophilus* NCFM | | Danisco | Shown in one study to reduce the side effects of antibiotic therapy. |
| *Lactobacillus paracasei* St11 (or NCC2461) | | | |
| *Lactobacillus johnsonii* La1 (= Lactobacillus LC1, *Lactobacillus johnsonii* NCC533) | | Nestlé | May reduce incidence of H pylori-caused gastritis and may reduce inflammation |
| *Lactobacillus plantarum* 299v | GoodBelly/ ProViva/ProbiMage | Probi | May improve symptoms of IBS; however, more research is required. |
| *Lactobacillus reuteri* American Type Culture Collection ATTC 55730 (*Lactobacillus reuteri* SD2112) | | BioGaia | Preliminary evidence for diarrhea mitigation in children, H. pylori infection, possible effect on gingivitis, fever in children and number of sick days in adults. |
| *Lactobacillus reuteri* Protectis (DSM 17938, daughter strain of ATCC 55730) | | | |
| *Saccharomyces boulardii* | DiarSafe and others | Wren Laboratories | Limited evidence for treatment of acute diarrhea. |
| *Lactobacillus rhamnosus* GR-1 *& Lactobacillus reuteri* RC-14 | Bion Flore Intime/ Jarrow Fem-Dophilus | Chr. Hansen | In one study, oral ingestion resulted in vaginal colonisation and reduced vaginitis. |
| *Lactobacillus acidophilus* NCFM *& Bifidobacterium bifidum* BB-12 | Florajen3 | American Lifeline, Inc | Preliminary evidence for reduced C. difficile-associated disease (CDAD). |
| *Lactobacillus acidophilus* CL1285 *& Lactobacillus casei* LBC80R | Bio-K+ CL1285 | Bio-K+ International | May affect digestive health. In vitro inhibition of *Listeria monocytogenes* and L. *innocua, Escherichia coli, Staphylococcus aureus, Enterococcus faecalis* and *Enterococcus faecium.* Reduction of symptoms of lactose intolerance and immune stimulation.^{[86]} |
| *Lactobacillus plantarum* HEAL 9 *& Lactobacillus paracasei* 8700:2 | Bravo Friscus/ ProbiFrisk | Probi | Is under study for common cold infections. |

Some additional forms of lactic acid bacteria, representing also suitable probiotics include:
- *Lactobacillus bulgaricus;*
- *Streptococcus thermophilus;*
- "*Lactobacillus bifidus" -* became new genus *Bifidobacterium.*

Some fermented products containing similar lactic acid bacteria include:
- Pickled vegetables
- Fermented bean paste such as tempeh, miso and doenjang;
- Kefir;
- Buttermilk or Karnemelk;
- Kimchi;
- Pao cai;
- Soy sauce;
- Zha cai.

### B. Prebiotics

In another embodiment of the present invention the dietary supplement compositions may include prebiotics. Prebiotics are defined as non-digestible food ingredients that may beneficially affect the host be selectively stimulating the growth and/or the activity of a limited number of bacteria in the colon. Adding prebiotics to the compositions leads to another inprovement of the stability of the anthocyanins against degradation within the intestine. The following describes in particular various oligosaccharides which can be taken into account as suitable prebiotics (component b2):
- ***Fructooligosaccharides.*** Fructooligosaccharides or FOS typically refer to short-chain oligosaccharides comprised of D-fructose and D-glucose, containing from three to five monosaccharide units. FOS, also called neosugar and short-chain FOS, are produced on a commercial scale from sucrose using a fungal fructosyltransferase enzyme. FOS are resistant to digestion in the upper gastrointestinal tract. They act to stimulate the growth of *Bifidobacterium* species in the large intestine. FOS are marketed in the United States in combination with probiotic bacteria and in some functional food products.
- ***Inulins.*** Inulins refer to a group of naturally-occurring fructose-containing oligosaccharides. Inulins belong to a class of carbohydrates known as fructans. They are derived from the roots of chicory *(Cichorium intybus)* and Jerusalem artichokes. Inulins are mainly comprised of fructose units and typically have a terminal glucose. The bond between fructose units in inulins is a beta-(2-1) glycosidic linkage. The average degree of polymerisation of inulins marketed as nutritional supplements is 10 to 12. Inulins stimulate the growth of *Bifidobacterium* species in the large intestine.
- ***Isomaltooligosaccharides.*** Isomaltooligosaccharides comprise a mixture of alpha-D-linked glucose oligomers, including isomaltose, panose, isomaltotetraose, isomaltopentaose, nigerose, kojibiose, isopanose and higher branched oligosaccharides. Isomaltooligosaccharides are produced by various enzymatic processes. They act to stimulate the growth of *Bifidobacterium* species and *Lactobacillus* species in the large intestine. Isomalto oligosaccharides are marketed in Japan as dietary supplements and in functional foods. They are being developed in the United States for similar uses.
- ***Lactilol.*** Lactilol is a disaccharide analogue of lactulose. Its pharmaceutical use is in the treatment of constipation and hepatic encephalopathy. Lactilol is also used in Japan as a prebiotic. It is resistant to digestion in the upper gastrointestinal tract and it is fermented by a limited number of colonic bacteria, resulting in an increase in the biomass of bifidobacteria and lactobacilli in the colon. Lactilol is known chemically as 4-0-(beta-D-galactopyranosyl)-D-glucitol. Lactilol is not approved for the treatment of hepatic encephalopathy or constipation in the U.S., and its use as a prebiotic is considered experimental. Lactilol is used in Europe as a food sweetener.
- ***Lactosucrose.*** Lactosucrose is a trisaccharide comprised of D-galactose, D-glucose and D-fructose. Lactosucrose is produced enzymatically by the enzymatic transfer of the galactosyl residue in lactose to sucrose. Lactosucrose is resistant to digestion in the stomach and small intestine. It is selectively utilized by intestinal*Bifidobacterium* species resulting in significant induction of growth of these bacteria in the colon. Therefore, under physiological conditions, lactosucrose acts on the intestinal microflora as a growth factor for *Bifidobacterium* species. Lactosucrose is also known as 4G-beta-D-galactosylsucrose. It is widely used in Japan as a dietary supplement and in functional foods, including yoghurt. Lactosucrose is being developed in the United States for similar uses.
- ***Lactulose.*** Lactulose is a semi-synthetic disaccharide comprised of the sugars D-lactose and D-fructose. The sugars are joined by a beta-glycosidic linkage, making it resistant to hydrolysis by human digestive enzymes. Lactulose is, however, fermented by a limited number of colonic bacteria. This can lead to changes in the colonic ecosystem in favour of bacteria, such as lactobacilli and bifidobacteria, which may confer some health benefits. Lactulose is a prescription drug in the United States for the treatment of constipation and hepatic encephalopathy. It is marketed in Japan for use as a dietary supplement and in functional foods. Its use in the United States as a prebiotic substance is still experimental.
- ***Pyrodextrins.*** Pyrodextrins comprise a mixture of glucose-containing oligosaccharides that is derived from the hydrolysis of starch. Pyrodextrins have been found to promote the proliferation of *Bifidobacterium* species in the large intestine. They are resistant to digestion in the upper gastrointestinal tract. Pyrodextrins are being developed for the nutritional supplement market place.
- *Soy **oligosaccharides.*** Soy oligosaccharides refer to oligosaccharides found in soybeans and also in other beans and peas. The two principal soy oligosaccharides are the trisaccharide raffinose and the tetrasaccharide stachyose. Raffinose comprises one molecule each of D-galactose, D-glucose and D-fructose. Stachyose consists of two molecules of D-galactose, one molecule of D-glucose and one molecule of D-fructose. Soy oligosaccharides act to stimulate the growth of *Bifidobacterium* species in the large intestine. They are marketed in Japan as dietary supplements and in functional foods. They are being developed in the United States for similar uses.
- ***Transgalactooligosaccharides.*** Transgalactooligosaccharides (TOS) are a mixture of oligosaccharides consisting of D-glucose and D-galactose. TOS are produced from D-lactose via the action of the enzyme beta-galactosidase obtained from *Aspergillus oryzae.* TOS are resistant to digestion in the upper gastrointestinal tract and stimulate the growth of bifidobacteria in the large intestine. TOS are marketed in Japan and Europe as dietary supplements and are used in functional foods. They are being developed for similar use in the United States.
- ***Xylooligosaccharides.*** Xylooligosaccharides are comprised of oligosaccharides containing beta (1? 4) linked xylose residues. The degree of polymerisation of xylooligosaccharides is from two to four. Xylo oligosaccharides are obtained by enzymatic hydrolysis of the polysaccharide xylan. They are marketed in Japan as prebiotics and are being developed for similar use in the United States.
- ***Biopolymers.*** Suitable biopolymers like e.g. beta-glucans include those originating from plants including cereals such as oats and barley, fungi, yeast, and bacteria. In addition, microbial cell wall preparations and whole cells rich in beta glucans are also suitable sources for beta glucan preparations useful for the present invention. Monomer residues in glucans can have 1-3 and 1-4, or 1-3 and 1-6 linkages (that is the monomer units are joined through 1,3, 1,4 or 1,6 bonds) and the percent of each type can vary. Preferably, beta glucans derived from yeast, particularly from *Saccharomyces,* preferably *Saccharomyces cerevisiae,* are used for the present invention. It will be appreciated, however, that other beta glucans would also be suitable. Further examples for suitable biopolymers are chitin and its derivatives, preferably oligoglucosamin and chitosan which represent a typical hydrocolloid.

### C. Vitamins

In another embodiment of the present invention the dietary supplement compositions may include vitamins (component b3). Vitamins have diverse biochemical functions. Some have hormone-like functions as regulators of mineral metabolism (e.g., vitamin D), or regulators of cell and tissue growth and differentiation (e.g., some forms of vitamin A). Others function as antioxidants (e.g., vitamin E and sometimes vitamin C). The largest number of vitamins (e.g., B complex vitamins) act as precursors for enzyme cofactors, that help enzymes in their work as catalysts in metabolism. In this role, vitamins may be tightly bound to enzymes as part of prosthetic groups: For example, biotin is part of enzymes involved in making fatty acids. Vitamins may also be less tightly bound to enzyme catalysts as coenzymes, detachable molecules that function to carry chemical groups or electrons between molecules. For example, folic acid carries various forms of carbon group ― methyl, formyl, and methylene ― in the cell. Although these roles in assisting enzyme-substrate reactions are vitamins' best-known function, the other vitamin functions are equally important. In the course of the present invention suitable vitamins are selected from the group consisting of
- Vitamin A (retinol, retinal, beta carotene),
- Vitamin B₁ (thiamine),
- Vitamin B₂ (riboflavin),
- Vitamin B₃ (niacin, niacinamide),
- Vitamin B₅ (panthothenic acid),
- Vitamin B₆ (pyridoxine, pyridoxamine, paridoxal),
- Vitamin B₇ (biotin),
- Vitamin B₉ (folic acid, folinic acid),
- Vitamin B₁₂ (cyanobalamin, hydoxycobalmin, methylcobalmin),
- Vitamin C (ascorbic acid),
- Vitamin D (cholecalciferol),
- Vitamin E (tocopherols, tocotrienols), and
- Vitamin K (phyolloquinone, menaquinone).

The preferred vitamins are ascorbic acid and tocopherols.

### D. Minerals

In another embodiment of the present invention the dietary supplement compositions may include minerals (component b4) that are selected from the group consisting of aluminium, antimony, arsenic, barium, beryllium, boron, bromide, cadmium, cerium, caesium, chloride, chrome, dysprosium, iron, erbium, europium, fluoride, gadolinium, gallium, germanium, gold, hafnium, holmium, indium, iridium, iodide, potassium, calcium, cobalt, copper, lanthanum, lithium, lutetium, magnesium, manganese, molybdenum, sodium, neodymium, nickel, niobium, osmium, palladium, phosphorus, platinum, praseodymium, rhenium, rhodium, rubidium, ruthenium, samarium, scandium, sulphur, selenium, silica, silver, strontium, tantalum, tellurium, terbium, thallium, thorium, thulium, titan, vanadium, ytterbium, yttrium, bismuth, wolfram, zinc, tin, zirconium, and their mixtures. The preferred mineral is zinc. The minerals can be added to the dietary supplement compositions in the form of their pharmaceutically acceptable salts.

### Dietary supplement compositions

The dietary supplement compositions according to the present invention may include
(a) about 0.5 to about 36 % b.w., preferably about 1 to about 25 % b.w. and more preferred about 2 to about 15 % b.w. of said anthocyanins, and
(b) 0 to about 15 % b.w., preferably about 0.5 to about 10 % b.w., and more preferred about 1 to about 8 % b.w. of at least one additive, for example selected from the groups (b1) to (b4) explained above.

The composition may represent a solid, a liquid or a capsule. For example it is possible to prepare an aqueous or alcoholic solution of the preparation that is subjected to lyophilisation or spray-drying to obtain a dry powder, a granule or a tablet. It is also possible to add such powder or an aqueous preparation directly to the final product, such as a beverage or a candy or a chewing gum. Finally it is possible to encapsulate the products.

### Encapsulation

The compositions are typically encapsulated by means of a solid covering material, which is preferably selected from starches, degraded or chemically or physically modified starches (in particular dextrins and maltodextrins), gelatins, gum arabic, agar-agar, ghatti gum, gellan gum, modified and non-modified celluloses, pullulan, curdlan, carrageenans, alginic acid, alginates, pectin, inulin, xanthan gum and mixtures of two or more of said substances.

The solid covering material is preferably selected from gelatin (preferred are pork, beef, chicken and/or fish gelatins and mixtures thereof, preferably comprising at least one gelatin with a bloom value of greater than or equal to 200, preferably with a bloom value of greater than or equal to 240), maltodextrin (preferably obtained from maize (corn), wheat, tapioca or potato, preferred maltodextrins have a DE value of 10 ― 20), modified cellulose (for example cellulose ether), alginates (for example Na-alginate), carrageenan (beta-, iota-, lambda- and/or kappa carrageenan), gum arabic, curdlan and/or agar-agar. Gelatin is preferably used, in particular, because of its good availability in different bloom values. Particularly preferred, especially for oral use are seamless gelatin or alginate capsules, the covering of which dissolves very rapidly in the mouth or bursts when chewing. Production may take place, for example, as described in EP 0389700 A1, US 4,251,195, US 6,214,376, WO 2003 055587 or WO 2004 050069 A1.

The capsules, however, may also represent micro-capsules. "Microcapsules" are understood to be spherical aggregates with a diameter of about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("microsponge") and, as microparticles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third etc. membrane. The membrane may consist of natural, semisynthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semisynthetic membrane materials are inter alia chemically modified celluloses, more particularly cellulose esters and ethers, for example cellulose acetate, ethyl cellulose, hydroxypropyl cellulose, hydroxypropyl methyl cellulose and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone.

Examples of known microcapsules are the following commercial products (the membrane material is shown in brackets) Hallcrest Microcapsules (gelatin, gum arabic), Coletica Thalaspheres (maritime collagen), Lipotec Millicapseln (alginic acid, agar agar), Induchem Unispheres (lactose, microcrystalline cellulose, hydroxypropylmethyl cellulose), Unicetin C30 (lactose, microcrystalline cellulose, hydroxypropylmethyl cellulose), Kobo Glycospheres (modified starch, fatty acid esters, phospholipids), Softspheres (modified agar agar) and Kuhs Probiol Nanospheres (phospholipids).

The active principles are released from the microcapsules by mechanical, thermal, chemical or enzymatic destruction of the membrane, normally during the use of the preparations containing the microcapsules. Despite the fact that the state of the art a huge range of possibilities for the encapsulation of actives, methods according to which a shell is obtained by coazervation, precipitation or polycondensation of anionic and cationic polymers has been quite suitable for the formation of stable capsules. Particularly, a preferred process for the encapsulation of active principles according to the present invention is characterised in that it comprises the steps of
(a) preparing a matrix from gel formers, cationic polymers and active principles;
(b) optionally dispersing said matrix in an oil phase; and
(c) treating said dispersed matrix with aqueous solutions of anionic polymers and optionally removing the in phase in the process.

Of course, anionic and cationic polymers in steps (a) and (c) can be exchanged.
(i) **Gel formers.** In the context of the invention, preferred gel formers are substances which are capable of forming gels in aqueous solution at temperatures above 40° C. Typical examples of such gel formers are heteropolysaccharides and proteins. Preferred thermogelling heteropolysaccharides are agaroses which may be present in the form of the agar agar obtainable from red algae, even together with up to 30% by weight of non-gel-forming agaropectins. The principal constituent of agaroses are linear polysaccharides of Galactose and 3,6-anhydro-L-galactose with alternate 1,3-and 1,4-glycosidic bonds. The heteropolysaccharides preferably have a molecular weight of 110,000 to 160,000 and are both odourless and tasteless. Suitable alternatives are pectins, xanthans (including xanthan gum) and mixtures thereof. Other preferred types are those which in 1% by weight aqueous solution still form gels that do not melt below 80° C. and solidify again above 40° C. Examples from the group of thermogelling proteins are the various gelatines.
(ii) **Anionic polymers.** Salts of alginic acid are preferred for this purpose. The alginic acid is a mixture of carboxyl-containing polysaccharides with the following idealized monomer unit: 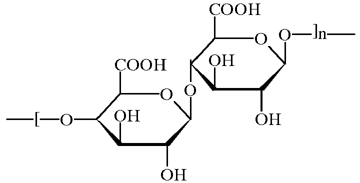 The average molecular weight of the alginic acid or the alginates is in the range from 150,000 to 250,000. Salts of alginic acid and complete and partial neutralization products thereof are understood In particular to be the alkali metal salts, preferably sodium alginate ("algin") and the ammonium and alkaline earth metal salts. Mixed alginates, for example sodium/magnesium or sodium/calcium alginates, are particularly preferred. In an alternative embodiment of the invention, however, carboxymethyl celluloses and anionic chitosan derivatives, for example the carboxylation and above all succinylation products are also suitable for this purpose.
(iii) **Cationic polymers.** Chitosans are biopolymers which belong to the group of hydrocolloids. Chemically, they are partly de-acetylated chitins differing in their molecular weights which contain the following ― idealized - monomer unit:

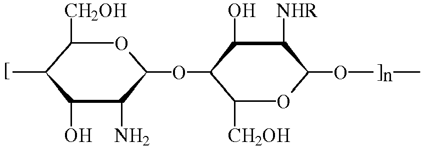

In contrast to most hydrocolloids, which are negatively charged at biological pH values, chitosans are cationic biopolymers under these conditions. The positively charged chitosans are capable of interacting with oppositely charged surfaces and are therefore used in cosmetic hair-care and body-care products and pharmaceutical preparations.

In a preferred embodiment of the invention a 1 to 10 and preferably 2 to 5% by weight aqueous solution of the gel former, preferably agar agar, is normally prepared and heated under reflux. A second aqueous solution containing the cationic polymer, preferably chitosan, in quantities of 0.1 to 2 and preferably 0.25 to 0.5% by weight and the active principle in quantities of 0.1 to 25 and preferably 0.25 to 10% by weight is added in the boiling heat, preferably at 80 to 100 ° C.; this mixture is called the matrix. Accordingly, the charging of the microcapsules with active principles may also comprise 0.1 to 25% by weight, based on the weight of the capsules. If desired, water-insoluble constituents, for example inorganic pigments, may also be added at this stage to adjust viscosity, generally in the form of aqueous or aqueous/alcoholic dispersions. In addition, to emulsify or disperse the active principles, it can be useful to add emulsifiers and/or solubilisers to the matrix. After its preparation from gel former, cationic polymer and active principle, the matrix optionally is very finely dispersed in an oil phase with intensive shearing in order to produce small particles in the subsequent encapsulation process. It has proved to be particularly advantageous in this regard to heat the matrix to temperatures in the range from 40 to 60° C while the oil phase is cooled to 10 to 20° C. The actual encapsulation, i.e. formation of the membrane by contacting the cationic polymer in the matrix with the anionic polymers, takes place in the third step. To this end, it is advisable to wash the matrix - dispersed in the oil phase - with an aqueous ca. 0.1 to 3 and preferably 0.25 to 0.5% by weight aqueous solution of the anionic polymer, preferably the alginate, at a temperature in the range from 40 to 100 and preferably 50 to 60° C. and, at the same time, to remove the oil phase if present. The resulting aqueous preparations generally have a microcapsule content of 1 to 10% by weight. In some cases, it can be of advantage for the solution of the polymers to contain other ingredients, for example emulsifiers or preservatives. After filtration, microcapsules with a mean diameter of preferably 1 to 3 mm are obtained. It is advisable to sieve the capsules to ensure a uniform size distribution. The microcapsules thus obtained may have any shape within production-related limits, but are preferably substantially spherical.

### Industrial application

Another object of the present invention is directed to either a juice or an extract comprising one or two anthocyanins, wherein the anthocyanin radical of said anthocyanins are essentially selected from the group consisting of
(a) Delphinidin and/or
(b) Cyanidin,
   and the corresponding sugar radicals are essentially selected from the group consisting of glucose, galactose, xylose, arabinose, rutinose, sophorose, sambubiose, or their mixtures,
   wherein
   (i) the content of disaccharides is less than about 2 % b.w. and
   (ii) optionally the content of potassium is about 10 to 5.000 ppm
      - both calculated on the composition - obtained from red berries or red blossoms, selected from the group consisting of selected from the group consisting of açai fruits, aronia, black and red currents, blackberries, black carrots, black tomatoes, blood oranges, blueberries, blackthorn, bog bilberries, cloudberries, cranberries, crowberries, elderberries, hibiscus, lingonberries, magellan barberries, maqui berries, mountain bilberries, prunes and prune plums, raspberries, red gooseberries, red grapes, rowanberries, sour cherries, strawberries, sweet cherries, tayberries or their mixtures.

Another embodiment of the present invention is directed to a non-therapeutic method for improving the health of a human by oral administration of a composition comprising the selected anthocyanins explained above. Finally, the present invention also encompasses the use of such composition for reducing cell damages caused by oxidation reactions or UV radiation.

### Examples

### A Extracts

For the following working examples a composition of anthocyanins obtained from *Aronia* (A) was prepared and compared with a commercial extract of *Vaccinium myrtillus* (B) obtained from Burgundy (Spain). The extracts were standardized to an anthocyanin concentration of about 25.5 % b.w. Analysis of the products was conducted by adding an internal standard (callistephin = pelargonidin chloride 3-glycoside from Carl Roth) and performing a reversed-phase solvent gradient HPLC using acetonitrile as the solvent. The anthocyanins were photometrical detected at a wavelength of 518 nm. The compositions and ratios by weight are compiled in Table C. It should be noted that Extract A is according to the invention, while Extract B serves for comparison.

**Table C**

| Composition of extracts (amounts in ppm) | | |
|---|---|---|
| **Compound** | **Extract A** | **Extract B** |
| Delphinidin-3-O-glucoside | | 8.010 |
| Delphinidin-3-O-galactoside | | 7.020 |
| Delphinidin-3-O-arabinoside | | 7.450 |
| Cyanidin-3-O-glucoside | 75.710 | 44.910 |
| Cyanidin-3-O-galactoside | 171.400 | 36.240 |
| Cyanidin-3-O-arabinoside | | 37.350 |
| Cyanidin-3-O-xyloside | 9.840 | |
| Petunidin-3-O-glucoside | | 42.040 |
| Petunidin-3-O-galactoside | | 38.660 |
| Petunidin-3-O-arabinoside | | 8.700 |
| Peonidin-3-O-glucoside | | 10.890 |
| Peonidin-3-O-galactoside | | 2.020 |
| Peonidin-3-O-arabinoside | | 1.950 |
| Malvidin-3-O-glucoside | | 2.320 |
| Malvidin-3-O-galactoside | | 6.220 |
| Malvidin-3-O-arabinoside | | 1.300 |
| **Total** | **256.950** | **255.080** |

### B Biochemical test methods

### Example 1, Comparative Examples C1 to C4

### Activity against free radicals

The effectiveness of the test substances against free radicals was tested by various chemical and biochemical methods:
- ***Method A***: A first test was carried out using diphenylpicrylhydrazyl (DPPH°) which is a relatively stable radical that forms a purple-coloured solution. The optical density (DO) at 513 nm was determined.
- ***Method B***: Hydroxyl radicals were released from hydrogen peroxide in the presence of iron (II) ions and EDTA and were used to oxidize desoxyribose. The oxidation product forms a pink-coloured compound with thiobarbituric acid. Its concentration corresponds to the optical density at 532 nm. A test was conducted to determine whether less desoxyribose is oxidized, i.e. whether fewer free radicals are released, in the presence of the test products.
- ***Method C***: The test just described was conducted in the absence of EDTA to determine the suitability of the test substances for forming inactive iron complexes.
- ***Method D***: Xanthine oxidase is an enzyme which is released through oxidative stress and catabolizes the decomposition of the purine bases adenine and guanine into uronic acid and superoxide anions. The superoxide anions dismute into hydrogen peroxide and oxygen either spontaneously or in the presence of Superoxid Dismutase. The quantity of superoxide anion can be determined by NBT reduction via the optical density at 490 nm. A test was conducted to determine whether fewer superoxide anions are generated or more anions are destroyed in the presence of the test substances.

The results are set out in **Table 1** and represent the EC50 values in % (w/v).

**Table 1**

| Activity against free radicals (%-rel.) | | | | | |
|---|---|---|---|---|---|
| **Ex.** | **Test product** | **Method** | | | |
| | | **A** | **B** | **C** | **D** |
| 1 | Extract A | 0.0010 | 0.25 | 0.85 | 0.45 |
| C1 | Extract B | 0.0024 | 0.26 | 0.94 | 0.51 |
| C2 | Tocopherol | 0.0067 | - | - | No effect |
| C3 | Ascorbic acid | 0.0013 | 0.26 | 0.9 | 0.59 |
| C4 | Butylhydroxytoluol | 0.0500 | - | - | No effect |

The results suggest that Extract A according to the present invention has anti-oxidative activity which is far superior to that of tocopherol and BHT and comparable with that of vitamin C. Compared to the standard bilberry Extract B the product according to the present invention also shows superior performance.

### Example 2, Comparative Examples C5 to C7

### Protection of cells against UVA radiation

The object of the following in vitro tests was to determine whether vaccinium extracts would protect human fibroblasts against oxidative stress and, more particularly, against the effects of UVA rays. UVA was selected as the stress factor because the rays penetrate into the dermis where they lead above all to lipoperoxidation of the cytoplasm membranes. The lipoperoxides formed are split into malonaldialdehydes (MDA) which are responsible for the crosslinking of many biomolecules such as, for example, proteins (enzyme inhibition) or nuclein bases (mutagenesis). To carry out the test, a fibroblast culture was mixed with foetal calf serum and, 2 days later, inoculated with the test substances. After incubation for 36 h at 37 °C and 5% by vol. CO₂, the nutrient medium was replaced by an electrolyte solution and the fibroblasts were damaged by a predetermined dose of UVA (3-15 J/cm 2). After the exposure, the quantity of MDA formed was determined in the supernatant solution by reaction with thiobarbituric acid while the content of proteins in the cell homogenisate was determined by Bradford's method. The results are set out in **Table 2** as %-rel against the standard. The table shows the mean value of two series of measurements involving triple determination.

**Table 2**

| Activity against UVA radiation (%-rel.) | | | | |
|---|---|---|---|---|
| **Ex.** | **Test product** | **Conc. [% w/v]** | **Liberated MDA** | **Cellular proteins** |
| C5 | Control without UVA | - | 0 | 101 |
| C6 | Control with UVA | - | 100 | 101 |
| C7 | Extract B | 0.004 | 84 | 93 |
| 2 | Extract A | 0.004 | 80 | 90 |

The results show that the Extract A according to the invention has a lastingly positive effect in combating oxidative stress without damaging the fibroblasts and is superior compared to the comparison Extract B.

### Example 3, Comparative Examples C8 to C10

### Protection of cells against UVB radiation

The function of this test was to show that Vaccinium extracts have anti-inflammatory properties for human keratinocytes. UVB was selected as the stress factor because the rays produce cutaneous inflammation (erythemas, oedemas) by activating enzymes that release arachidonic acid, such as phospholipase A2 (PLA2) for example. This results not only in damage to the membranes, but also in the formation of inflammatory substances, such as prostaglandins of the PGE2 type for example. The influence of UVB rays on keratinocytes was determined in vitro through the release of cytoplasmatic enzymes, such as LDH (lactate dehydrogenase) for example, which runs parallel to the cell damage and the formation of PGE2. To carry out the test, a fibroblast culture was mixed with foetal calf serum and inoculated with the test substances 2 days later. After incubation for 36 h at 37 °C and a CO₂ level of 5% by vol., the nutrient medium was replaced by an electrolyte solution and the fibroblasts were damaged with a particular dose of UVB (50 mJ/cm 2). The quantity of keratinocytes was determined after trypsination via a cell counter while the LDH concentration was enzymatically determined. The results are set out in Table 3 which shows the activity in %-rel. against a standard as the mean value of two test series involving double determination.

**Table 3**

| Activity against UVB radiation (%-rel.) | | | | | |
|---|---|---|---|---|---|
| **Ex.** | **Test product** | **Conc. [% w/v]** | **Cellular DNA** | **Liberated LDH** | **Liberated PGE2** |
| C8 | Control without UVB | | 100 | 0 | 0 |
| C9 | Control with UVB | | 31 | 100 | 100 |
| C10 | Extract B | 0.005 | 64 | 88 | 75 |
| C3 | Extract A | 0.005 | 60 | 85 | 70 |

The results show that Extract A significantly reduces the harmful effects of UVB rays and, in particular, reduces the release of LDH and PGE2, and shows a better performance compared to the comparison Extract B

## Claims

1. A dietary supplement composition comprising one or two anthocyanins, wherein the anthocyanin radical of said anthocyanins are essentially selected from the group consisting of
(a) Delphinidin and/or
(b) Cyanidin,
and the corresponding sugar radicals are essentially selected from the group consisting of glucose, galactose, xylose, arabinose, rutinose, sophorose, sambubiose, or their mixtures,
wherein
(i) the content of disaccharides is less than about 2 % b.w. and
(ii) optionally the content of potassium is about 10 to 5.000 ppm
- both calculated on the composition.

2. The composition of Claim 1, wherein said anthocyanins are obtained from juices and/or extracts from red berries or red blossoms, selected from the group consisting of açai fruits, aronia, black and red currents, blackberries, black carrots, black tomatoes, blood oranges, blueberries, blackthorn, bog bilberries, cloudberries, cranberries, crowberries, elderberries, hibiscus, lingonberries, magellan barberries, maqui berries, mountain bilberries, prunes and prune plums, raspberries, red gooseberries, red grapes, rowanberries, sour cherries, strawberries, sweet cherries, tayberries or their mixtures.

3. The composition of Claim 1, wherein they also encompass at least one phenolic acid selected from the group consisting of gallic acid, sinapic acid, procatechuic acid, cholorogenic acid and/or their esters.

4. The composition of Claim 1, wherein in case delphinidin and cyanidin are both present their the ratio by weight is about 1 : 3 to about 3 : 1.

5. The composition of Claim 1, comprising
(a) anthocyanins, and
(b) at least one additive selected from the group consisting of (b1) probiotic micro-organisms, (b2) prebiotics, (b3) vitamins and (b4) minerals.

6. The composition of Claim 5, wherein said probiotic micro-organisms forming sub-group (b1) are selected from lactic acid bacteriae and/or bifidobacteriae.

7. The composition of Claim 5, wherein said probiotics forming sub-group (b2) are selected from fructooligosaccharides, inulins, isomaltooligosaccharides, lactilol, lactosucrose, lactulose, pyrodextrins, soy oligosaccharides, transgalactooligosaccharides, xylooligosaccharides, biopolymers and their mixtures.

8. The composition of Claim 5, wherein said vitamins forming sub-group (b3) are selected from Vitamin A (retinol, retinal, beta carotene), Vitamin B₁ (thiamine), Vitamin B₂ (riboflavin), Vitamin B₃ (niacin, niacinamide), Vitamin B₅ (panthothenic acid), Vitamin B₆ (pyridoxine, pyridoxamine, paridoxal), Vitamin B₇ (biotin), Vitamin B₉ (folic acid, folinic acid), Vitamin B₁₂ (cyanobalamin, hydoxycobalmin, methylcobalmin), Vitamin C (ascorbic acid), Vitamin D (cholecalciferol), Vitamin E (tocopherols, tocotrienols), and Vitamin K (phyolloquinone, menaquinone).

9. The composition of Claim 5, wherein said minerals forming sub-group (b4) are selected from the group consisting of aluminium, antimony, arsenic, barium, beryllium, boron, bromide, cadmium, cerium, caesium, chloride, chrome, dysprosium, iron, erbium, europium, fluoride, gadolinium, gallium, germanium, gold, hafnium, holmium, indium, iridium, iodide, potassium, calcium, cobalt, copper, lanthanum, lithium, lutetium, magnesium, manganese, molybdenum, sodium, neodymium, nickel, niobium, osmium, palladium, phosphorus, platinum, praseodymium, rhenium, rhodium, rubidium, ruthenium, samarium, scandium, sulphur, selenium, silica, silver, strontium, tantalum, tellurium, terbium, thallium, thorium, thulium, titan, vanadium, ytterbium, yttrium, bismuth, wolfram, zinc, tin, zirconium and their mixtures.

10. The composition of Claim 5, comprising
(a) about 0.5 to about 36 % b.w. anthocyanins and
(b) 0 to about 15 % b.w. of at least one additive.

11. The composition of Claim 7 representing a solid, a liquid or a capsule.

12. A juice comprising one or two anthocyanins, wherein the anthocyanin radical of said anthocyanins are essentially selected from the group consisting of
(a) Delphinidin and/or
(b) Cyanidin,
and the corresponding sugar radicals are essentially selected from the group consisting of glucose, galactose, xylose, arabinose, rutinose, sophorose, sambubiose, or their mixtures,
wherein
(i) the content of disaccharides is less than about 2 % b.w. and
(ii) optionally the content of potassium is about 10 to 5.000 ppm
- both calculated on the composition - obtained from red berries or red blossoms, selected from the group consisting of selected from the group consisting of açai fruits, aronia, black and red currents, blackberries, black carrots, black tomatoes, blood oranges, blueberries, blackthorn, bog bilberries, cloudberries, cranberries, crowberries, elderberries, hibiscus, lingonberries, magellan barberries, maqui berries, mountain bilberries, prunes and prune plums, raspberries, red gooseberries, red grapes, rowanberries, sour cherries, strawberries, sweet cherries, tayberries or their mixtures.

13. An extract comprising one or two anthocyanins, wherein the anthocyanin radical of said anthocyanins are essentially selected from the group consisting of
(a) Delphinidin and/or
(b) Cyanidin,
and the corresponding sugar radicals are essentially selected from the group consisting of glucose, galactose, xylose, arabinose, rutinose, sophorose, sambubiose, or their mixtures,
wherein
(i) the content of disaccharides is less than about 2 % b.w. and
(ii) optionally the content of potassium is about 10 to 5.000 ppm
- both calculated on the composition - obtained from red berries or red blossoms, selected from the group consisting of selected from the group consisting of açai fruits, aronia, black and red currents, blackberries, black carrots, black tomatoes, blood oranges, blueberries, blackthorn, bog bilberries, cloudberries, cranberries, crowberries, elderberries, hibiscus, lingonberries, magellan barberries, maqui berries, mountain bilberries, prunes and prune plums, raspberries, red gooseberries, red grapes, rowanberries, sour cherries, strawberries, sweet cherries, tayberries or their mixtures.

14. A non-therapeutic method for improving the health of a human by oral administration of a composition according to Claim 1.

15. The use of a composition according to Claim 1 for reducing cell damages caused by oxidation reactions or UV radiation.
